# EUROPEAN PATENT APPLICATION

(11) **EP 4 837 497 A1**
(43) Date of publication of application: **11.11.2026**
(21) Application number: 25175459.4
(22) Date of filing: 09.05.2025
(51) Int. Cl.: A61B 1/00

(54) **COUPLING DEVICE FOR ROTATABLY COUPLING AN INSERTION TUBE TO A BODY OF A SURGICAL DEVICE**

(71) Applicant: PENTAX Medical Corporation, Akishima-shi, Tokyo 196-0012 (JP)
(72) Inventor: WALBERG, Erik, Tokyo, 160-8347 (JP)
(74) Representative: Lucke, Andreas

(57) **Abstract**

Disclosed herein is a coupling device (200) for rotatably coupling an insertion tube (104) to a body (102) of a surgical device (100, 300) and a surgical device comprising such a coupling device. The coupling device comprises a body-side part (202) that is attached or attachable to the body of the surgical device and a tube-side part (204) that is attached or attachable to the insertion tube. The tube-side part is configured to rotatably engage the body-side part so as to allow for rotation of the insertion tube relative to the body of the surgical device and so as to retain the insertion tube at one or more discrete rotational orientations relative to the body of the surgical device.

## Description

### FIELD OF THE DISCLOSURE

The present disclosure is in the field of medical technology. In particular, the present disclosure relates to a coupling device for rotatably coupling an insertion tube to a body of a surgical device and to a surgical device, in particular an endoscope, including such a coupling device.

### BACKGROUND

An endoscope is a surgical device that may be used to access (e.g., view, extract or treat) tissue within the body of a patient by inserting one or more medical tools (instruments) into the body through an orifice of the body. The endoscope may include (i.e., comprise) an interface/control body and an insertion tube that is coupled to the interface/control body. The insertion tube is configured to be inserted into the body of the patient and may include one or more channels to provide access to tissue within the body. The one or more channels may, e.g., be configured to receive a medical tool and/or a fluid and to guide the medical tool and fluid, respectively, to the tissue of interest. Examples of such medical tools include imaging probes for viewing tissue and biopsy tools for extracting tissue samples.

To access the tissue of interest within the body of the patient, the insertion tube may have to be maneuvered precisely within the patient's body. Conventional endoscopes typically involve a fixed attachment between the insertion tube and the interface/control body of the endoscope, which can limit the flexibility and range of motion required for accessing the tissue of interest. This fixed attachment can make it challenging for users (e.g., surgeons, physicians or other medical practitioners) to navigate the insertion tube through complex anatomical pathways and to precisely position or orient the insertion tube relative to the tissue of interest, potentially leading to less effective diagnostic and/or therapeutic outcomes. Users may have to manually rotate the entire interface/control body to achieve the desired positioning or orientation of the insertion tube. Such handling of the endoscope may involve a significant amount of movement (e.g., rotation) of the body, arm and/or wrist of the user as well as potentially awkward body posturing, which may induce fatigue of the user.

To overcome these disadvantages, endoscopes with rotatable insertion tubes have been proposed such as for example in US 10,182,701 B2 and US 7,833,153 B2. Known solutions may, however, require complex coupling mechanisms involving a large number of parts. Moreover, a precise positioning of the insertion tube relative to the interface/control body may be cumbersome and time-consuming.

### SUMMARY OF THE DISCLOSURE

It is thus an object of the present disclosure to provide an improved mechanism for rotatably coupling an insertion tube to an interface/control body of an endoscope that facilitates precise rotational control of the insertion tube relative to the interface/control body with reduced complexity of the coupling mechanism.

This object is met by a coupling device for rotatably coupling an insertion tube to a body of a surgical device and a surgical device including such a coupling device as set out in the independent claims. Examples thereof are detailed in the dependent claims.

The present disclosure provides a coupling device for rotatably coupling an insertion tube to a body of a surgical device. The coupling device includes a body-side part that is attached or attachable to the body of the surgical device and a tube-side part that is attached or attachable to the insertion tube. The tube-side part is configured to rotatably engage the body-side part so as to allow for rotation of the insertion tube relative to the body of the surgical device. The tube-side part is further configured to rotatably engage the body-side part so as to retain the insertion tube at one or more discrete rotational orientations relative to the body of the surgical device.

In the context of the present disclosure, terms relating to spatial directions, positions and/or orientations may, unless mentioned otherwise or implied by context otherwise, refer to directions and positions, respectively, relative to the insertion tube (when coupled to the body of the surgical device via the coupling device). For example, the longitudinal axis and the longitudinal (or axial) direction (of the insertion tube) may refer to the axis and direction, respectively, along the insertion tube, i.e., along the length of the insertion tube (e.g., in the direction from a proximal end of the insertion tube to a distal end of the insertion tube or vice-versa). The term "distal" as used herein may refer to an element, a feature or a location (e.g., along the length of the tube) that is to be closer to a location of tissue being accessed within the body of a patient during use of the surgical device (e.g. facing in the direction of the tissue being accessed and/or being further away from the body of the surgical device), whereas the term "proximal" as used herein may refer to an element, a feature or a location (e.g., along the length of the tube) that is to be closer to a user (e.g., a surgeon, a physician or other medical practitioner) during use of the surgical device (e.g. facing away from the tissue being accessed and/or being closer to the body of the surgical device). The (longitudinal) direction extending from the proximal end of the insertion tube to the distal end may be referred to as the "distal direction" and the (longitudinal) direction from the distal end of the insertion tube to the proximal end may be referred to as the "proximal direction". A direction along a circumference of the tube (for example parallel to a surface, e.g. an outer surface, of the tube and perpendicular to the longitudinal direction) may be referred to as a circumferential, azimuthal or angular direction. A direction perpendicular to a surface, e.g. an outer surface, of the tube (for example perpendicular to the longitudinal direction and to the circumferential direction) may be referred to as a radial direction. The longitudinal, circumferential and radial directions may for example define a cylindrical coordinate system. Depending on the state or configuration of the tube, the orientation of these directions may vary along the length of the tube, e.g. as a result of a bending of the tube (i.e., the tube and thus the longitudinal direction may follow a bent or curved path). With regard to the coupling device and/or the body of the surgical device, these directions may refer to the respective directions at a proximal portion or end of the insertion tube (when coupled to the body) or the respective directions when extended (in the proximal direction) beyond the proximal end of the insertion tube.

The coupling device may be configured to couple (e.g., connect and/or attach) the insertion tube (e.g., a proximal portion or end thereof) to the body (e.g., a distal portion or end thereof) of the surgical device such that the insertion tube can be rotated relative to the body, for example about an axis (e.g., a longitudinal axis or direction) of the insertion tube. The coupling device may for example be embodied as a connector (e.g., an adapter) for connecting the insertion tube to the body. The coupling device may be configured to couple the insertion tube to the body such that the insertion tube (e.g., one or more channels thereof) is in fluid communication with the body (e.g., one or more inlets thereof). The insertion tube is not particularly limited and may be any insertion tube as used for surgical devices, in particular endoscopes, e.g., as described below. The body of the surgical device may for example be an interface and/or control body of the surgical device, e.g., as described below.

The body-side part and the tube-side part may each be embodied as a single piece or as a multi-piece assembly. The body-side part may be permanently or removably attached (or attachable) to the body, e.g., to a distal portion or end thereof. In some examples, the body-side part may be provided as part of the body of the surgical device (e.g., integrally formed with the body or with a part thereof), for example as detailed below. The tube-side part may be permanently or removably attached (or attachable) to the insertion tube, e.g., to a proximal portion or end thereof. In some examples, the tube-side part may be provided as part of the insertion tube (e.g., integrally formed with the insertion tube or with a part thereof).

The tube-side part is configured to engage the body-side part, e.g., to be arranged on and/or in the body-side part, for coupling the insertion tube to the body of the surgical device. The tube-side part may be configured to be connected (e.g., attached) to the body-side part, for example so as to attach the insertion tube to the body of the surgical device. The tube-side part may be configured to engage the body-side part such that the tube-side part (and thereby the insertion tube) is axially retained (e.g., fixed) relative to the body-side part, i.e., such that the tube-side part cannot move relative to the body-side part in the axial direction or can only move within a certain (very) limited range of motion.

The coupling device is configured to allow for rotation of the insertion tube relative to the body of the surgical device (when the insertion tube is coupled to the body of the surgical device via the coupling device). For this, the tube-side part is configured to rotatably engage (e.g. be supported or held rotatably by) the body-side part such that the tube-side part (and thereby the insertion tube) can rotate relative to the body-side part (and thereby relative to the body of the surgical device). The body-side part (e.g., a bearing feature such as a bearing surface or structure supporting the tube-side part) may define an axis of rotation for the tube-side part (and thereby the insertion tube), which may be aligned with the longitudinal axis of the insertion tube.

The tube-side part may be configured to rotatably engage the body-side part via a snap fit, e.g., via one or more interlocking members on the tube-side part configured to snap into and/or onto (e.g., interlock with) corresponding counterpart(s) on the body-side part and/or vice-versa. The tube-side part may for example be configured to be connected to the body-side part by pushing or pressing (e.g., snapping or clicking) the tube-side part onto and/or into the body-side part or vice versa.

One (or both of) of the body-side part and the tube-side part may include a (respective) cantilever configured to snap into and/or onto (e.g., interlock with) a corresponding receiving structure on the other one of the body-side part and the tube-side part, e.g., such that the insertion tube is axially retained relative to the body of the surgical device. The receiving structure may for example include a pair of retaining features (e.g., retaining surfaces or structures) that are configured to come in contact with the cantilever (e.g., a projection such as a nose or pin thereon) and prevent the cantilever (and thereby the respective one of the body-side part and tube-side part) from moving beyond the respective retaining feature in the proximal and distal direction, respectively. The receiving structure may for example be or include a groove, wherein the groove may, e.g., be configured to receive (and axially retain) a corresponding counterpart on the cantilever (e.g., a projection thereon).

The coupling device is configured to retain (e.g., azimuthally retain) the insertion tube at one or more discrete rotational orientations (e.g., angular or azimuthal orientations) relative to the body of the surgical device (when the insertion tube is coupled to the body of the surgical device via the coupling device). The coupling device may be configured to retain the insertion tube at a single discrete rotational orientation (e.g., a reference orientation such as a center orientation at a center of a range of rotation of the insertion tube) or at a plurality of discrete rotational orientations (e.g., at least three, in some examples at least five, in one example at least ten orientations). The plurality of discrete rotational orientations may for example extend (e.g., be distributed) over an angular range of between 30° and 360°, in some examples of between 60° and 270°, in one example of between 90° and 240°.

As used herein, the term "retaining" an element relative to another element may refer to holding or keeping the element in place, wherein the element may for example be held or kept in place such that the element cannot move (e.g., rotate) spontaneously (e.g., as a result of forces or torques as they typically occur spontaneously or accidentally during operation of the surgical device unless deliberately moving the element) relative to the other element while still allowing for the element to be moved (e.g., rotated) relative to the other element deliberately by applying a sufficiently high force or torque). In other words, the coupling device is configured such that a user can manually rotate the insertion tube relative to the body to a respective one of the discrete rotational orientation(s) and the coupling device will retain (e.g., hold or keep) the insertion tube at the respective rotational orientation afterwards (e.g., during subsequent operation of the surgical device and/or until the user rotates the insertion tube relative to the body to a different rotational orientation). The coupling device may for example be configured to retain the insertion tube at each of the one or more discrete rotational orientations up to a torque of at least 0.1 Nm, in some examples up to a torque of at least 0.5 Nm, in some examples up to a torque of at least 1.0 Nm, in one example up to a torque of at least 2.0 Nm, in one example up to a torque of at least 5 Nm and in one example up to a torque of at least 10 Nm (i.e., a torque in excess of the aforementioned limits may be required for rotating the insertion tube out of a discrete rotational orientation).

As used herein, the term "discrete rotational orientation" may refer to a rotational orientation (e.g., an azimuthal orientation with respect to/about the axial direction) of a finite set of distinct rotational orientations, i.e., rotational orientations that can be numbered or indexed and distinguished from other rotational orientations (and from each other). A discrete rotational orientation may in particular be a rotational orientation that is structurally distinct from other rotational orientations, e.g., defined by one or more structural features of the coupling device. Put differently, from a discrete rotational orientation, the insertion tube may not continuously transition to other rotational orientations (as, e.g., in a continuous rotation of cylindrical surfaces on each other) but may be structurally confined to the respective discrete rotational orientation (in both directions of rotation). Around each of the discrete rotational orientation(s), the coupling device may be configured to generate a restoring force driving the insertion tube towards the respective rotational orientation. Some or all of the discrete rotational orientation(s) may be distinct from the end points of a range of rotation of the insertion tube (i.e., may lie within the range of rotation and may not be associated with an end stop limiting the range of rotation of the insertion tube, noting that an end stop, apart from preventing further rotation of the insertion tube, typically does not retain the insertion tube in the sense that the insertion tube is free to move back in the opposite direction).

One of the body-side part and the tube-side part may include one or more protrusions and/or one or more depressions. The protrusion(s) and/or depression(s) may be arranged (e.g., distributed) along a circumference of the body-side part and the tube-side part, respectively, e.g., along the azimuthal direction around the axis of rotation. The protrusion(s) and/or depression(s) may be configured to engage a corresponding counterpart (e.g., a depression and a protrusion, respectively) on the other one of the body-side part and the tube-side part, for example so as to retain the insertion tube at said one or more discrete rotational orientations relative to the body of the surgical device. For example, the receiving structure (e.g., the groove) for the cantilever may include one or more protrusions and/or one or more depressions that are configured to engage a corresponding counterpart on the cantilever. The protrusion(s) and/or the depression(s) may for example be arranged at a bottom of the groove and/or at a sidewall of the groove.

In some examples, the protrusions and/or depressions form a toothed feature (e.g., a toothed surface or structure) that defines a plurality of discrete rotational orientation for the insertion tube relative to the body of the surgical device. The toothed feature may for example be similar to a gear or cog wheel (or a circular segment thereof).

The coupling device may further include one or more deformable members that are arranged between the body-side part and the tube-side part (e.g., between bearing surfaces of the body-side part and the tube-side part). The one or more deformable members may be configured to generate friction resisting the rotation of the insertion tube relative to the body of the surgical device. The one or more deformable members may be compressed (e.g., squeezed or pressed together) by the body-side part and/or the tube-side part (e.g., the bearing surfaces thereof). The one or more deformable members may include or consist of an elastic material, e.g., an elastomer or rubber. In one example, one or more of the deformable members are embodied as O-rings. One or more of the deformable members may be configured to provide a seal between the body-side part and the tube-side part (e.g., to seal an interface between the body-side part and the tube-side part), for example to prevent fluid from escaping between the body-side part and the tube-side part.

One or more of the deformable members may be arranged between axial bearing surfaces of the body-side part and of the tube-side part. The axial bearing surfaces may extend perpendicular or substantially perpendicular to the axial direction (i.e., the axis) of the insertion tube (e.g., at an angle between 70° and 110°, in some examples between 80° and 100°, in one example between 85° and 95° to the axial direction). The axial bearing surfaces may axially compress said one or more of the deformable members (i.e., in a direction parallel or substantially parallel to the axial direction). Additionally or alternatively, one or more of the deformable members may be arranged between radial bearing surfaces of the body-side part and of the tube-side part. The radial bearing surfaces may extend parallel or substantially parallel to the axial direction of the insertion tube (e.g., at an angle between -20° and +20°, in some examples between -10° and +10°, in one example between -5° and +5° to the axial direction). The radial bearing surfaces may radially compress said one or more of the deformable members (i.e., in a direction perpendicular or substantially perpendicular to the axial direction).

The coupling device may include one or more end stops (e.g., mechanical end stops) configured to limit a range of rotation of the insertion tube relative to the body of the surgical device. The end stop(s) may be arranged on one or both of the body-side part and the tube-side part and/or on one or both of the body of the surgical device and the insertion tube. The end stop(s) may be configured to come in contact with a corresponding counterpart (e.g., on the other one of the body-side part and the tube-side part) when the insertion tube reaches a respective endpoint (rotational orientation) so as to prevent further rotation of the insertion tube beyond the endpoint. The range of rotation of the insertion tube may for example be between 30° and 360°, in some examples between 60° and 270°, in one example between 90° and 240°. The end stop(s) may be associated with one of the discrete rotational orientations (e.g., such that the respective endpoint corresponds to one of the rotational orientation) or may not be associated with any of the discrete rotational orientations (e.g., such that the respective endpoint is (azimuthally) offset from each of the respective rotational orientation).

The body-side part may be integrally formed with the body of the surgical device, for example with a frame or a body cover (e.g., a housing or casing) of the body of the surgical device. In other words, the body-side part may not be provided as a separate piece that is attached or to be attached to the body, but may be integrally formed therewith as a single piece, which may facilitate assembly of the surgical device.

The tube-side part may include a kink protection for preventing kinking of the insertion tube at the body of the surgical device (e.g., at or adjacent to a distal end of the body and/or at or adjacent to the body-side part). The kink protection may surround the insertion tube (e.g., a proximal portion or a part thereof), e.g., extend around the circumference of the insertion tube. The kink protection may extend along the insertion tube in the distal direction, e.g., from the tube-side part and/or from the body of the surgical device. The kink protection may be made of (i.e., include or consist of) a more rigid material than the insertion tube (e.g., a rigid or semi-rigid material) to prevent kinking of the insertion tube. The kink protection may for example be made of an elastomer or rubber.

Additionally or alternatively, the tube-side part may include a grip for rotating the insertion tube relative to the body of the surgical device. The grip may for example have a structure and/or textured surface (e.g., one or more protrusions and/or depressions) and/or may be or include a gripping member such as a handle to facilitate handling (e.g., rotating) the tube-side part. In some examples, the grip may be made of a same material as the kink protection and may in particular be integrally formed with the kink protection as a single piece (i.e., the grip and the kink protection may not be formed as separate pieces that are then connected or attached to each other or the tube-side part).

Additionally or alternatively, the tube-side part may include a rotator that is attached or attachable to the insertion tube and configured to rotatably engage the body-side part. The rotator may be rotationally fixed to the insertion tube, i.e. such that the insertion tube rotates with the rotator when the rotator is rotated relative to the body-site part. The rotator may be formed as a single piece or as a multi-piece assembly. The tube-side part may further include a cover that is arranged or arrangeable over the rotator for covering and operating the rotator. For operating the rotator, the cover may be keyed or otherwise rotationally fixed to the rotator, e.g., by one or more interlocking members. The cover may be formed as a single piece or as a multi-piece assembly. In some examples, the cover (e.g., as an integrally formed single piece) forms one or both of the aforementioned kink protection and the aforementioned grip.

In any of the examples described herein, the coupling device may optionally include a locking mechanism configured to lock the body-side part and the tube-side part relative to each other so as to prevent rotation of the insertion tube relative to the body of the surgical device. As used herein, the term "locking" an element relative to another element may refer to preventing any motion of the element relative to the other element (when applying forces or torques as typically applied during operation of the surgical device, including for rotation of the insertion tube relative to the body of the surgical device). In other words, the force or torque that is necessary for rotating the insertion tube out of a discrete rotational orientation (at which the insertion tube is retained) to another (e.g., discrete) rotational orientation may be lower, preferably substantially lower (e.g., less than 50%, in some examples less than 20%, in one example less than 10% and in one example less than 5%) than the force or torque that would be required to rotate the insertion tube relative to the body of the surgical device when the body-side part and the tube-side part are locked by the locking mechanism. Additionally or alternatively, the locking mechanism may be configured to lock the body-side part and the tube-side part relative to each other so as to prevent rotation of the insertion tube relative to the body of the surgical device up to a torque of at least 1.0 Nm, in some examples up to a torque of at least 2.0 Nm, in some examples up to a torque of at least 5 Nm, in some examples up to a torque of at least 10 Nm, in one example up to a torque of at least 20 Nm, in one example up to a torque of at least 50 Nm and in one example up to a torque of at least 100 Nm.

The locking mechanism may be configured to lock the body-side part and the tube-side part relative to each other by friction (for example by (e.g., further) compressing one or more of the deformable members arranged between the body-site part and the tube-site part). Additionally or alternatively, the locking mechanism may be configured to lock the body-side part and the tube-side part relative to each other by interlocking members (e.g., mechanical stops) on the body-site part, the tube-site part, the body of the surgical device and/or the insertion tube.

The present disclosure further provides a surgical device including an interface and/or control body, an insertion tube and a coupling device according to any one of the examples described herein for rotatably coupling the insertion tube to the interface and/or control body.

The surgical device may in particular be an endoscope, for example a bronchoscope, a uteroscope, a colonoscope, a duodenoscope or a gastrointestinal endoscope. In other examples, the surgical device may, e.g., be a laparoscope (i.e., a surgical device for accessing tissue within the body of a patient through an (artificial) incision in the body, whereas an endoscope may be for accessing tissue within the body of a patient through a (natural) orifice of the body). In some examples, the surgical device may be a single-use ("disposable") device, e.g., a surgical device that is configured for a single use and intended to be thrown away after use (e.g., not configured or suitable for a second use, e.g., for disinfection or sterilization).

The interface and/or control body (which in short may also be referred to as interface/control body herein) may for example be or include a grip or handle for operating the surgical device. The interface/control body may be used to interface to and/or control the insertion tube of the surgical device and/or an instrument inserted into the insertion tube. The interface/control body may for example include one or more inlets in fluid communication with the insertion tube (when coupled thereto via the coupling device), e.g., to provide a medical tool (e.g., a biopsy tool) and/or a fluid to the tube. The interface/control body may, e.g., include one or more control features such as knobs or wheels, which may for example be used for navigating (e.g., bending) and/or fixing (e.g., braking) the insertion tube (e.g., an active bending section thereof) and/or for operating the instrument inserted into the tube.

The insertion tube is not particularly limited and may be any insertion tube as used in surgical devices and in particular endoscopes. The insertion tube may for example include one or more passively bendable sections (passive bending sections) and/or one or more actively bendable sections (active bending sections). A passively bendable section may, e.g., be formed of a bendable or flexible material or structure, whereas an actively bendable section may, e.g., include one or more joints that can be articulated (e.g., using one or more control wires) for actively bending the insertion tube. The insertion tube may include one or more channels to provide access to tissue within the body, e.g., at least a working channel configured to receive a medical tool (instrument) such as a biopsy tool and/or an imaging probe and, optionally, one or more additional channels, for example a fluid channel (e.g., for rinsing). In some examples, the insertion tube may include, e.g., in a distal tip thereof, an imaging probe, which may for example include an image collection device such as a photodetector (e.g., a camera sensor) or entrance facet(s) of one or more optical waveguides. Additionally or alternatively, the insertion tube may include, e.g., a distal tip thereof, an illumination source such as a light source (e.g., one or more light-emitting diodes) or exit facet(s) of one or more optical waveguides.

The coupling device of the surgical device may include a locking mechanism configured to lock the body-side part and the tube-side part relative to each other, e.g., as described above. The interface and/or control body may include a lock control feature for operating the locking mechanism, for example to transition the locking mechanism from a locked (or closed) state, in which the body-side part and the tube-side part are locked relative to each other, to an unlocked (or open) state, in which the body-site part and the tube-side part are not locked relative to each other (e.g., free to rotate relative to each other and, optionally, retained at one of the discrete rotational orientations), and vice-versa. The lock control feature may for example be or include a lever or button configured to operate the locking mechanism (e.g., via an actuating member such as an actuating wire or rod).

Additionally or alternatively, the interface/control body may include an angulation control feature for operating an active bending section of the insertion tube, for example via one or more control wires. The angulation control feature may be configured to bend the active bending section, e.g., by articulating one or more joints in the active bending section. The angulation control feature may be configured to bend the active bending section in one plane (e.g., back and forth in one direction), or preferably in two (e.g., orthogonal) planes (e.g., back and forth in a first direction and back and forth in a second direction different from, e.g., perpendicular to, the first direction).

The lock control feature may be arranged adjacent to the angulation control feature so as to allow for single-handed operation of both the locking mechanism and the active bending section (e.g., simultaneously and/or without repositioning of the hand of the user). For example, a distance between the lock control feature and the angulation control feature may be less than 15 cm, preferably less than 10 cm, in one example less than 5 cm. The lock control feature and/or the angulation control feature may be arranged in a proximal portion of the interface/control body.

In some examples, the coupling device of the surgical device includes said locking mechanism configured to lock the body-side part and the tube-side part relative to each other (e.g., as described above) and the interface and/or control body includes a lock control feature for operating the locking mechanism (e.g., as described above) and an angulation control feature for operating an active bending section of the insertion tube (e.g., as described above), said lock control feature being arranged adjacent to said angulation control feature so as to allow for single-handed operation of both the locking mechanism and the active bending section.

Additionally or alternatively, the lock control feature may be arranged adjacent (e.g., within the aforementioned distances) to an inlet of the interface/control body, in particular an instrument inlet for inserting an instrument into the insertion tube such as a biopsy inlet for a biopsy tool. This may for example allow for single-handed operation of both the locking mechanism and the instrument (e.g., inserting the instrument into the insertion tube and/or using the instrument).

### LIST OF FIGURES

In the following, a detailed description of the present disclosure and examples thereof is given with reference to the figures. The figures show schematic illustrations of
Fig. 1: a surgical device according to an example of the present disclosure;
Fig. 2a and 2b: a coupling device for rotatably coupling an insertion tube to a body of a surgical device in accordance with an example of the present disclosure;
Figs. 3a to 3e: an endoscope with a coupling device according to an example of the present disclosure;
Fig. 4a to 4c: a coupling device for rotatably coupling an insertion tube to a body of a surgical device providing a plurality of discrete rotational orientations in accordance with an example of the present disclosure;
Fig. 5a and 5b: a coupling device for rotatably coupling an insertion tube to a body of a surgical device with an axially compressed deformable member in accordance with an example of the present disclosure;
Fig. 6a and 6b: a coupling device for rotatably coupling an insertion tube to a body of a surgical device with a radially compressed deformable member in accordance with an example of the present disclosure; and
Fig. 7a and 7b: a coupling device for rotatably coupling an insertion tube to a body of a surgical device with a pair of axially compressed deformable members in accordance with an example of the present disclosure.

### DESCRIPTION OF EXAMPLES

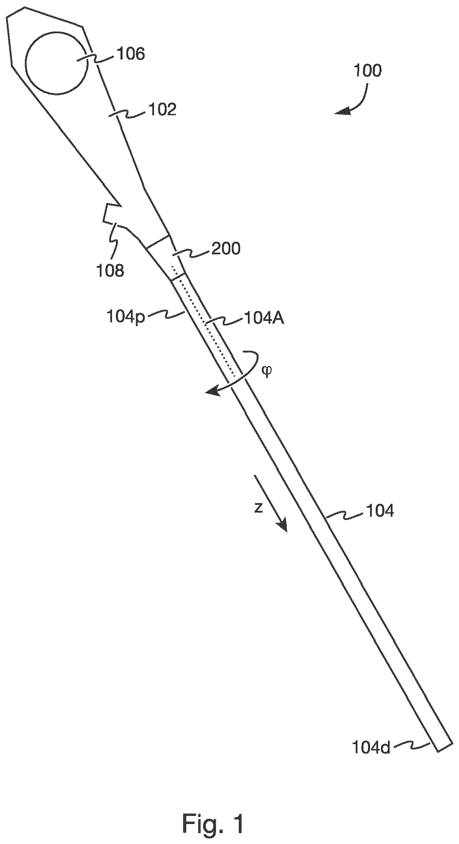

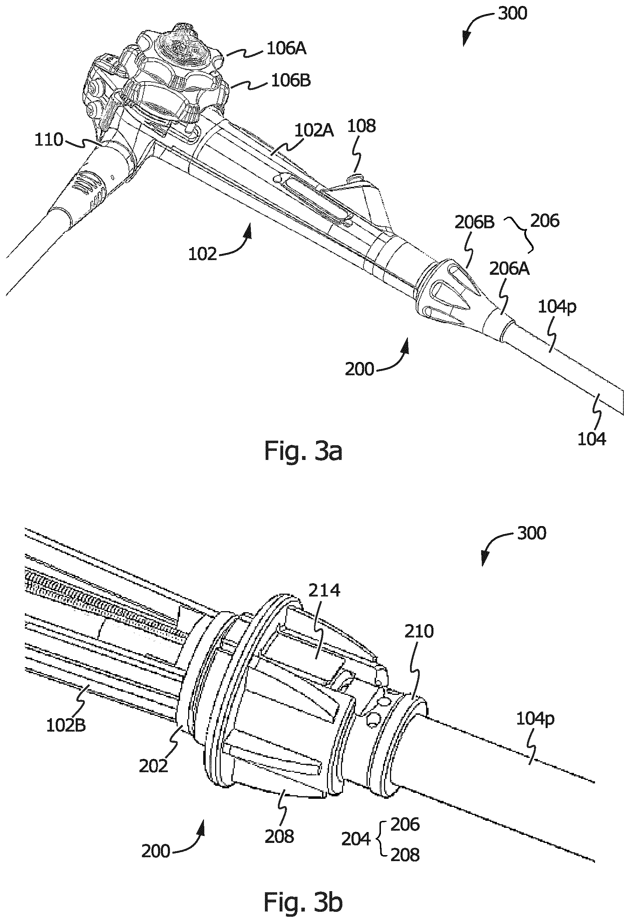

Fig. 1 shows a schematic illustration (not to scale) of a surgical device 100 according to an example of the present disclosure. The surgical device 100 may for example be an endoscope (e.g., similar to the endoscope 300 of Figs. 3a-e) or a laparoscope. The surgical device 100 includes an interface and/or control body (interface/control body) 102, to which an insertion tube 104 is coupled via a coupling device 200.

The interface/control body 102 includes one or more control features 106 such as knobs or wheels for operating the surgical device 100 (e.g., for operating the insertion tube 104). The one or more control features 106 may for example include an angulation control feature for operating an active bending section (not shown) of the insertion tube 104, e.g., as detailed below for the endoscope 300 of Fig. 3a. The interface/control body 102 further include one or more inlets 108, e.g., for receiving an instrument such as a biopsy probe and/or for receiving and/or extracting a fluid.

The insertion tube 104 extends from a proximal portion 104p adjacent (and coupled) to the interface/control body 102 to a distal portion or tip 104d facing away from the interface/control body 102. The direction along which the insertion tube 104 extends (illustrated by the arrow labelled Z in Fig. 1) is referred to as the axial or longitudinal direction herein (noting that, depending on the state of the insertion tube 104, the insertion tube 104 and thus the longitudinal direction may follow a bent or curved path), wherein the direction from the proximal portion 104p to the distal tip 104d is referred to as the distal direction and the direction from the distal tip 104d to the proximal portion 104p is referred to as the proximal direction. The insertion tube 104 (e.g., a center or center line thereof) defines an axis 104A which extends along the longitudinal direction and, in the proximal portion 104p, is indicated by the correspondingly labelled dotted line in Fig. 1. The direction around a circumference of the insertion tube 104 (around the axis 104A) is referred to as the circumferential, azimuthal or angular direction and indicated by the arrow labelled φ in Fig. 1. The direction perpendicular to the longitudinal and azimuthal direction (e.g., perpendicular to an outer surface of the insertion tube 104 and parallel to the direction of view in Fig. 1) is referred to as the radial direction.

The insertion tube 104 is configured to be inserted into the human or animal body (e.g., through an incision or orifice) and to provide access to tissue within the body. For this, the insertion tube 104 may include a (main) working channel (not shown) and, optionally, one or more additional channels (not shown), for example a fluid channel for rinsing. The channels may be used for inserting (e.g., receiving and guiding) one or more instruments (such as a biopsy tool and/or an imaging probe, not shown) and/or one or more fluids into the body. The one or more inlets 108 on the interface/control body 102 may be in fluid communication with the insertion tube 104 (via the coupling device 200), e.g., with the working channel in the insertion tube 104.

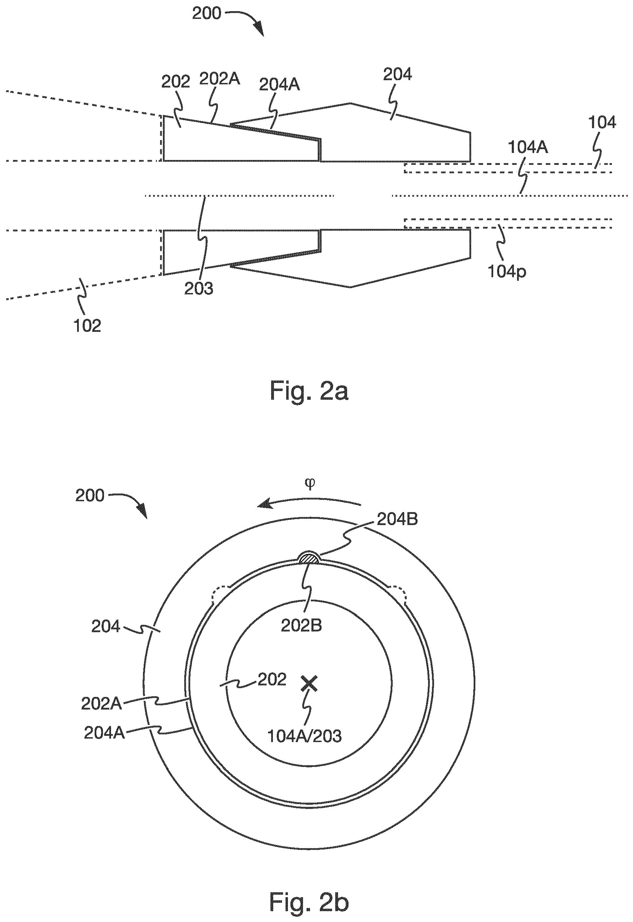

The coupling device 200 rotatably couples the insertion tube 104 to the interface/control body 102 such that the insertion tube 104 can be rotated relative to the interface/control body 102 about its axis 104A (i.e., in the azimuthal direction φ). The coupling device 200 may be a coupling device according to any one of the examples described herein and may for example be embodied as detailed below with reference to Figs. 2 to 7, for example as the coupling device 200 of Figs. 2a and 2b described in the following.

Figs. 2a and 2b depict a schematic illustration (not to scale) of a coupling device 200 for rotatably coupling an insertion tube 104 to a body 102 of a surgical device (e.g., the surgical device 100 of Fig. 1). The coupling device 200 is shown in a cross-sectional side view (e.g., along the radial direction) in Fig. 2a and in a cross-sectional front view (e.g., along the proximal direction) in Fig. 2b.

The coupling device 200 includes a body-side part 202 and a tube-side part 204. The body-side part 202 is configured to be (e.g., removably) attached to a distal end of the body 102 such that the body-side part 202 is rotationally fixed (i.e., does not rotate) relative to the body 102. In some examples, the body-side part 202 may be part of the body 102 and may in particular be integrally formed with the body 102 (or a part thereof), for example as detailed below with reference to Figs. 3a-e. The tube-side part 204 is configured to be (e.g., removably) attached to a proximal portion 104p of the insertion tube 104 such that the tube-side part 204 is rotationally fixed (i.e., does not rotate) relative to the insertion tube 104.

The tube-side part 204 rotatably engages the body-side part 202 such that the tube-side part 204 (and thereby the insertion tube 104) can be rotated relative to the body-side part 202 (and thereby the body 102). The body-side part 202 (e.g., a surface thereof such as the outer surface 202A serving as a bearing surface for a surface such as the inner surface 204A of the tube-side part 204), which is rotationally fixed to the body 102, defines an axis of rotation 203 for the tube-side part 204 (and thereby the insertion tube 104). Preferably, the axis of rotation 203 coincides with the axis 104A of the insertion tube 104, i.e. such that the insertion tube 104 can be rotated around its axis 104 (in the azimuthal direction).

The device 200 is configured to retain the tube-side part 204 (and thereby the insertion tube 104) at at least one discrete rotational orientation relative to the body-side part 202 (and thereby relative to the body 102). For this, a protrusion 202B is provided on the outer surface 202A of the body-side part 202. The protrusion 202B is configured to engage (e.g., be received in) a corresponding counterpart such as a depression (e.g., a recess or cutout) 204B on the inner surface 204A of the tube-side part 204 as illustrated in Fig. 2b. When the protrusion 202B is received in the depression 204B, the protrusion 202B may prevent the tube-side part 204 from spontaneously or accidentally rotating relative to the body-side part 202, thereby retaining the tube-side part 204 at the (discrete) rotational orientation defined by the corresponding structures (i.e., the protrusion 202B and the depression 204B). Additionally or alternatively, the tube-site part 204 may also be retained at the discrete rotational orientation by friction, e.g., between the surfaces 202A and 204A and/or between the surfaces 202A, 204A and a deformable member such as an O-ring arranged therebetween. The discrete rotational orientation may be a reference orientation at which the insertion tube 104 has a predetermined azimuthal orientation relative to the body 102, for example a center orientation at a center of the range of rotation of the insertion tube 104.

The device 200 (e.g., the protrusion 202B and the depression 204B) is configured such that the tube-side part 204 may be rotated out of the discrete rotational orientation (at which the protrusion 202B is received in the depression 204B) by applying a sufficiently large force or torque to the tube-side part 204 and/or the insertion tube 104 (e.g., overcoming the restoring force on the protrusion 202B generated by the depression 204B and/or overcoming the friction). The protrusion 202B and/or the surface 204A may for example be deformable (e.g., made of an elastic material). Additionally or alternatively, the protrusion 202B may be movable in the radial direction, e.g., arranged on an elastic member such as a cantilever, for example as detailed below with reference to Fig. 3a-e. This may allow for arranging the tube-site part 204 (and thereby the insertion tube 104) at other rotational orientations, e.g., at any of the continuum of rotational orientations at which the protrusion 202B does not engage the depression 204B ("continuous rotational orientations"). When rotating the tube-site part 204 out of (or across) the discrete rotational orientation, the device 200 (e.g., the protrusion 202B and the depression 204B) may provide a haptic and/or acoustic feedback, for example a noticeable resistance, bump and/or click.

Optionally, the device 200 may be configured to retain the tube-side part 204 at a plurality of discrete rotational orientations relative to the body-sized part 202. For example, instead of a single depression 204B, a plurality of counterparts (e.g., depressions) for the protrusion 202B may be provided on the inner surface 204A of the tube-side part 204 distributed along the circumference of the tube-side part 204 (e.g., the depression 204B and two or more additional depressions as illustrated in dotted lines in Fig. 2b). Each of the plurality of counterparts may define a respective discrete rotational orientation at which the tube-site part 204 may be retained. Additionally or alternatively, a plurality of protrusions may be provided on the outer surface 202A of the body-site part 202, each defining respective discrete rotational orientation. The plurality of discrete rotational orientations may for example be arranged equidistantly (in the azimuthal direction), e.g., every 10°, every 20° or every 30°.

The device 200 may further be configured to maintain (e.g., hold) the tube-side part 204 (and thereby the insertion tube 104) at other rotational orientations different from the discrete rotational orientation(s) defined by the protrusion 202B and the depression(s) 204B, e.g., at one of the continuous rotational orientations at which the protrusions 202B does not engage any of the depression(s) 204B (but is, e.g., arranged between adjacent depression(s) 204B). The device 200 may for example be configured to maintain the tube-side part 204 at these other rotational orientations by friction, for example between the protrusion 202B and the surface 204A, between the surfaces 202A and 204A and/or between the surfaces 202A, 204A and a deformable member such as an O-ring arranged therebetween. Put differently, the friction may be sufficiently large such that the tube-side part 204 does not spontaneously or accidentally rotate relative to the body-side part 202. As compared to the discrete rotational orientations defined by the protrusion(s) 202B and the depression(s) 204B, there may be no restoring force towards the other rotational orientations and/or the force or torque required for moving the body-site part 204 out of one of the other rotational orientations may the smaller, in some examples substantially smaller than the force or torque required for moving the body-side part out of one of the discrete rotational orientations.

Fig. 3a shows a surgical device 300 in accordance with another example of the present disclosure. The surgical device 300 is similar to the surgical device 100 of Fig. 1 and also includes an interface and/or control body 102, to which an insertion tube 104 is rotatably coupled via a coupling device 200. In the example of Fig. 3a, the surgical device 300 is embodied as an endoscope (e.g., a bronchoscope or a duodenoscope).

The interface/control body 102 of endoscope 300 has an internal frame 102B (see Fig. 3b) arranged in a body cover 102A (e.g., a housing or casing). The interface/control body 102 includes a pair of angulation control features 106A, 106B for operating an active bending section (not shown) of the insertion tube 104, e.g., by articulating one or more joints in the active bending section via one or more control wires. The angulation control feature 106A may for example be configured to bend the active bending section in a first plane, whereas the angulation control feature 106B may be configured to bend the active bending section in a second plane perpendicular to the first plane (thus, e.g., allowing for two-dimensional control of the distal tip of the insertion tube 104).

Similar to the surgical device 100 of Fig. 1, the interface/control body 102 includes one or more inlets 108, for example at least an instrument inlet such as a biopsy inlet. The inlets 108 may be in fluid communication with the insertion tube 104 via the coupling device 200. The interface/control body 102 may further include one or more connectors 110, which may for example be configured to provide fluid, electrical and/or optical connections to the interface/control body 102 and, optionally, to the insertion tube 104 via the interface/control body 102 (e.g., to a channel outlet and/or an imaging probe at the distal tip 104d of the insertion tube 104).

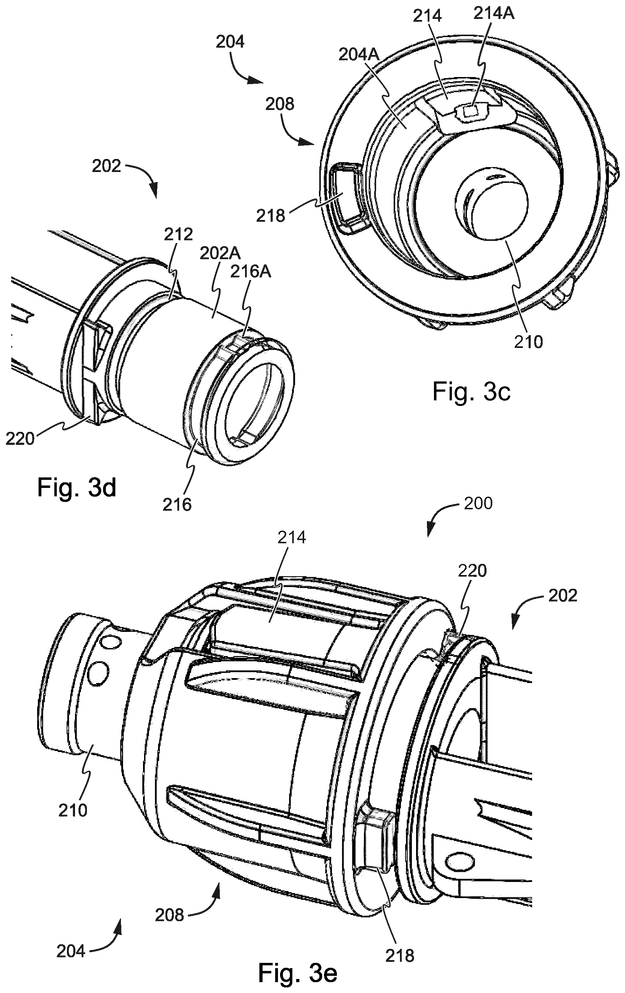

The coupling device 200 is similar to the coupling device of Figs. 2a and 2b and also includes a body-side part 202 and a tube-side part 204 for rotatably coupling the insertion tube 104 to the interface/control body 102, e.g., as described above. Figs. 3b and 3e show close-up views of the coupling device 200 (with the cover 206 and the body cover 102A removed), Fig. 3c shows a close-up view of the tube-side part 204 of the coupling device 200 (with the cover 206 removed) and Fig. 3d of the body-side part 202 of the coupling device 200 (with the body cover 102A removed).

The tube-side part 204 (see, e.g., Figs. 3a and 3c) includes an (optional) cover 206 and a rotator 208 that is covered by the cover 206, e.g., so as to not the visible and/or accessible from the outside. The cover 206 is rotationally fixed relative to rotator 208 (e.g., keyed thereto) such that the rotator 208 can be operated (e.g., rotated) via the cover 206. The cover 206 includes a kink protection 206A that extends in the distal direction and surrounds the proximal portion 104p of the insertion tube 104 so as to prevent the proximal portion 104p of the insertion tube 104 from kinking at the interface to the interface/control body 102 (e.g., at the rotator 208). The cover 206 further includes a grip 206A for operating the rotator 208. The grip 206A may for example include a structured and/or textured surface, e.g., one or more protrusions and/or depressions as illustrated in Fig. 3, to facilitate operation of the rotator 208. The cover 206, i.e. kink protection 206A and the grip 206B may be integrally formed as a single piece, for example from an elastomer or rubber. In some examples, the tube-side part 204 may not include the cover 206 but may, e.g., be formed by the rotator 208 only.

The rotator 208 includes an attachment portion 210 that is configured to receive the proximal portion 104p of the insertion tube 104. The attachment portion 210 may be configured to be attached (and rotationally fixed) to the proximal portion of the insertion tube 104 by any suitable fastening means, for example by a press fit, a snap fit and/or screws, and/or adhesive. The rotator 208 is configured to rotatably engage (e.g., be arranged on and rotate about) the body-side part 202, for example such that an inner surface 204A of the rotator 208 rotates about (e.g., on) an outer surface 202A of the body-side part 202, thereby allowing for rotating the insertion tube 104 relative to the interface/control body 102 (e.g., as described above with reference to Figs. 2a and 2b).

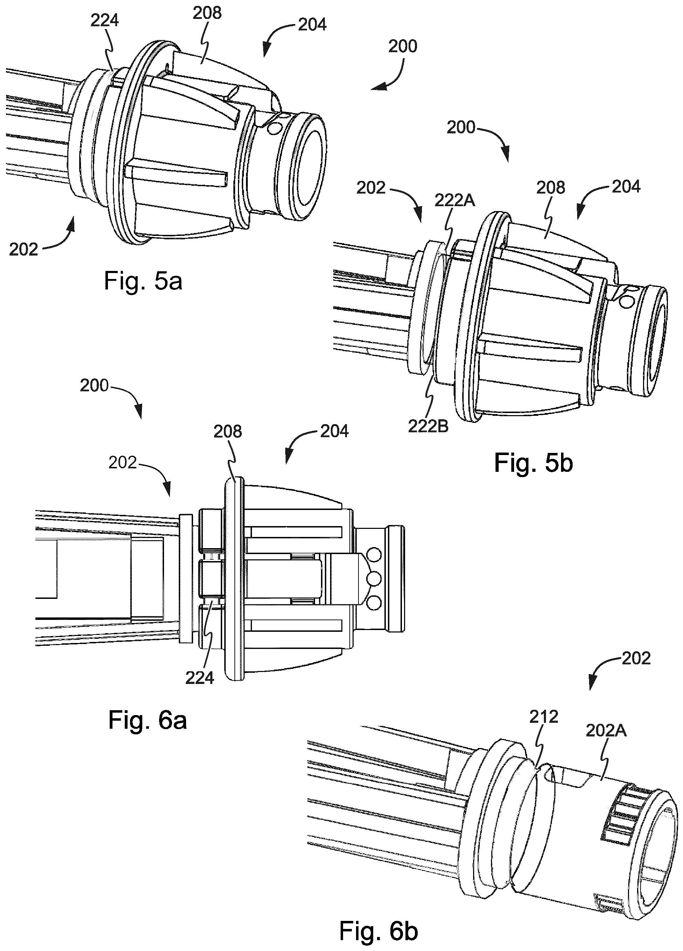

In the example of Figs. 3a-e, the body-side part 202 is integrally formed with the interface/control body 102, for example with the frame 102B thereof. The body-side part 202, e.g., the outer surface 202A thereof, may be provided with a groove 212 extending around the circumference of the body-side part 202 as illustrated in Fig. 3d. In the groove 212 (and, optionally in a corresponding groove or on an approximately cylindrical surface on the inner surface 204A of the rotator 208), a deformable member (not shown) may be arranged to generate friction resisting the rotation of the rotator 208 on the body-side part 202, e.g., as detailed below with reference to Figs. 5 to 7.

The tube-side part 204 (e.g., the rotator 208) is configured to engage (e.g., be connected to) the body-side part 202 via a snap fit. In the example of Figs. 3a-e, the tube-side part 204 (e.g., the rotator 208) is provided with an elastic cantilever 214 having a projection 214A at its tip. The projection 214A is configured to snap into a corresponding receiving structure, namely a groove 216, on the body-side part 202, thereby axially retaining the tube-side part 204 relative to the body-side part 202 (e.g., by the projection 214A being or coming in contact with the sidewalls of the groove 216, which may extend perpendicular or substantially perpendicular to the rotation axis/the axis of the insertion tube 104). The tube-side part 204 can thus be connected to the body-side part 202 by simply pressing (e.g., snapping or clicking) the tube-side part 204 (e.g., the rotator 208) onto the body-side part 202. This may facilitate assembly of the endoscope 300, which may be particularly advantageous for single-use endoscopes, where one or both of the body 102 and the insertion tube 104 are designed as disposable items that are not to be re-used.

The snap fit between the tube-side part 204 and the body-side part 202 is configured such that the tube-side part 204 remains connected to the body-side part 202 during operation of the endoscope 300 (e.g., does not move in the axial direction at forces as they typically occur during operation of the endoscope 300). The snap fit may further be configured to allow for removal of the tube-side part 204 from the body-side part 202 (e.g., by applying a sufficiently large force in the axial direction and/or manually opening the cantilever 214), for example to replace the insertion tube 104 or for cleaning of the insertion tube 104 and/or of the body 102.

The groove 216 is configured such that the projection 214A of the cantilever 214 can move (e.g., slide) along the groove 216 to allow for rotation of the rotator 208 about the body-side part 202. The groove 216 may for example have a flat bottom. In some examples, the groove 216 may extend along the entire circumference of the body-side part 202. Along the groove 216, a pair of protrusions 216A is arranged (e.g., on the bottom of the groove 216 as illustrated in Fig. 3d or, additionally or alternatively, on one or both sidewalls of the groove 216). The protrusions 216A form a depression (e.g., a recess) therebetween that is configured to receive and retain the projection 214 on the cantilever 214. Thereby, the tube-side part 204 with the insertion tube 104 can be retained at a particular (discrete) rotational orientation, which may, e.g., correspond to a center of a range of rotation of the tube-side part 204/the insertion tube 104. The range of rotation may for example be limited by a pair of end stops 220 that are configured to come in contact with a projection 218 on tube-side part 204 (e.g., the rotator 208) so as to prevent the tube-side part 204 from rotating beyond a corresponding endpoint. The cantilever 214 is configured (e.g., sufficiently elastic) such that the projection 214A can be moved over the protrusions 216A so as to rotate the tube-side part 204 away from the discrete rotational orientation. Moving the projection 214 across one of the protrusions 216 may produce a noticeable feedback for a user of the endoscope 300, e.g., a haptic and/or acoustic feedback such as a tactile and/or audible click.

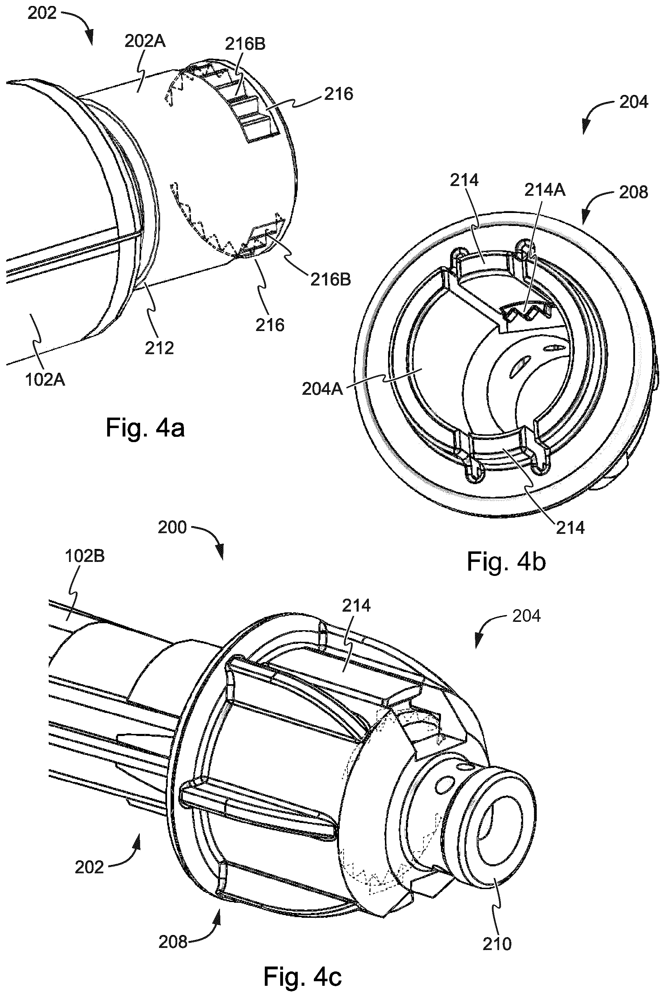

In some examples, the tube-side part 204 (e.g., the rotator 208) may include a second cantilever similar to the cantilever 214, which may for example be arranged opposite to (e.g., rotated by 180° from) the cantilever 214, e.g., as described below with reference to Figs. 4a-4c. Additionally or alternatively, the groove 216 may include a second pair of protrusions similar to the protrusions 216A, which may for example be arranged opposite to (e.g., rotated by 180° from) the protrusions 216A, e.g., as described below with reference to Figs. 4a-4c.

Figs. 4a-c show schematic illustrations of a coupling device 200 for rotatably coupling an insertion tube to a body of a surgical device (such as the surgical devices 100 and/or 300) according to another example of the present disclosure. The coupling device 200 is similar to the coupling device in Figs. 3a-e and also includes a tube-side part 204 that rotatably engages a body-side part 202, e.g., as described above. Fig. 4a shows the body-side part 202 of the coupling device 200, Fig. 4b the tube-side part 204 and Fig. 4c the tube-side part 204 connected to the body-side part 202.

The coupling device 200 of Figs. 4a-c differs from the coupling device of Figs. 3a-e in that, instead of the pair of protrusions 216, a toothed feature 216B formed by a plurality of protrusions/teeth is arranged at the bottom of the groove 216. The toothed feature 216B is configured to retain the cantilever 214 on the body-side part 204 (e.g., on the rotator 208 thereof) at a plurality of discrete positions. The toothed feature 216 thus defines a plurality of discrete rotational orientations at which the insertion tube 104 (not shown), when attached to the tube-side part 204, can be retained. The plurality of discrete rotational orientations may for example include between 5 and 20 rotational orientations. The discrete rotational orientations may be arranged equidistantly along the azimuthal direction, e.g., spaced apart by between 2° to 30° each. To aid retainment of the cantilever 214 by the toothed feature 216B, the projection 214A may also be provided with a corresponding toothed feature as illustrated in Fig. 4b (instead of being formed as a single protrusion or nose as in Fig. 3c).

The groove 216 may extend around the entire circumference of the body-side part 202 or, as in the example of Figs. 4a-c, may only partially extend around the circumference of the body-side part 202. End faces of the groove 216 (which may, e.g., extend parallel or substantially parallel to the rotation axis/the axis of the insertion tube) may form mechanical stops limiting the range of rotation of the tube-side part 204 and the insertion tube 104 (e.g., by coming in contact with end faces of the projection 214A). In some examples, the end faces of the groove 216 may not be aligned with one of the discrete rotational orientations but may, e.g., be displaced therefrom (i.e., the end position may not correspond to one of the discrete rotational orientations).

In some examples, the tube-side part 204 (e.g., the rotator 208) may be provided with a pair of cantilevers 214 arranged on opposite sides (along the azimuthal direction) of the tube-side part 204 as illustrated in Fig. 4b. Correspondingly, the body-side part 202 may be equipped with a pair of grooves 216 (each with a respective toothed feature 216B at its bottom) on opposite sides of the body-side part 202 as illustrated in Fig. 4a. This may facilitate retaining the insertion tube 104 at the discrete rotational orientations and/or may provide a smoother bearing or guiding of the tube-side part 204 on the body-side part 202. In some examples, the tube-side part 204 may include a cover (not shown) covering the tube-side part 204 (e.g., the rotator 208) similar to Figs. 3a-e.

Figs. 5a, 5b show schematic illustrations of a coupling device 200 for rotatably coupling an insertion tube to a body of a surgical device (such as the surgical devices 100 and/or 300) according to another example of the present disclosure. The coupling device 200 is similar to the coupling device in Figs. 3a-e and also includes a tube-side part 204 that rotatably engages a body-side part 202, e.g., as described above. A deformable member 224, which in the example of Figs. 5a, 5b is embodied as an O-ring, is arranged between axial bearing surfaces 222A and 222B on the body-side part 202 and the tube-side part 204, respectively. The axial bearing surfaces 222A, 222B are normal to the axis of rotation of the tube-side part 204 (e.g., normal to the axis of the insertion tube, not shown) and may squeeze or compress the deformable member 224 therebetween in the axial direction. The deformable member 224 introduces friction between the body-side part 202 and the tube-side part 204, which must be overcome in order to rotate the tube-side part 204 relative to the body-side part 202. This friction may retain or aid in retaining the tube-side part 204 at the discrete rotational orientation defined by the protrusions 216A or alternatively the toothed feature 216B (not visible in Figs. 5a, 5b). Additionally or alternatively, the friction may allow for maintaining the tube-side part 204 at other (e.g., continuous/non-discrete) rotational orientations, e.g., within the continuum of rotational orientations where the projection 214A on the cantilever 214 is arranged on the flat bottom of the groove 216 (i.e., away from the protrusions 216A). For example, the friction may prevent the tube-side part 204 from rotating relative to the body-side part 202 unless deliberately actuated even when the projection 214 is not retained by the protrusions 216A (but could, e.g., freely slide along the flat bottom of the groove 216). The amount of friction may depend on (and thus be set or adjusted by) an amount of pressure exerted on the deformable member 224 by the bearing surface 222A, 222B. For example, the stronger the deformable member 224 is squeezed or compressed, the larger the amount of friction generated by the deformable member 224 may be. This may allow for adjusting the amount of friction as needed (e.g., so as to reliably retain or maintain the tube-side part 204 azimuthally) and for implementing a locking mechanism for locking the body-side part 202 and the tube-side part 204 relative to each other (e.g., by axially moving the tube-side part 204 relative to the body-side part 202 so as to increase or decrease the pressure exerted on the deformable member 224, for example via a lock control feature on the body 102 coupled to the body-side part 202 via an actuating member such as an actuating wire or rod).

Figs. 6a, 6b show schematic illustrations of a coupling device 200 for rotatably coupling an insertion tube to a body of a surgical device (such as the surgical devices 100 and/or 300) according to another example of the present disclosure. The coupling device 200 is similar to the coupling device in Figs. 5a, 5b except that in this example, the deformable member 224 is arranged (e.g., squeezed or compressed) between radial bearing surfaces that are parallel to the axis of rotation of the tube-side part 204 (e.g., parallel to the axis of the insertion tube, not shown), namely between the outer surface 202A of the body-side part 202 and the inner surface of the tube-side part 204 (e.g., similar to the inner surface 204A of the tube-side part of Figs. 3a-e). The outer surface 202A is provided with an annular groove 212 extending around the circumference of the outer surface 202A, in which the deformable member 224 (e.g., again embodied as an O-ring) is arranged. A similar groove (not visible) may be provided on the inner surface 204A of the tube-side part 204 for arranging the deformable member 224 therein.

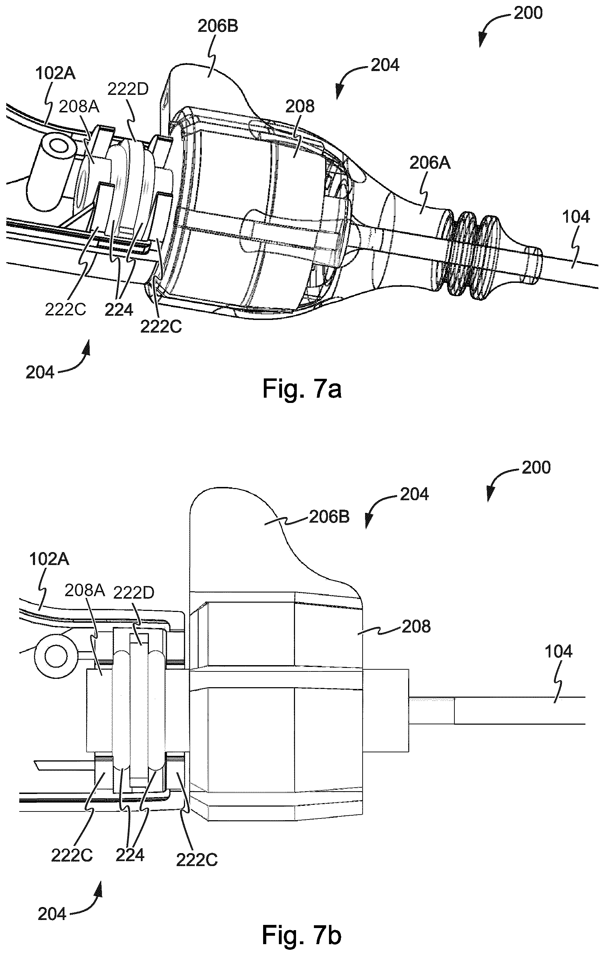

Figs. 7a, 7b show schematic illustrations of a coupling device 200 for rotatably coupling an insertion tube to a body of a surgical device (such as the surgical devices 100 and/or 300) according to another example of the present disclosure. The coupling device 200 is similar to the coupling device of Figs. 3a-e and also includes a tube-side part 204 that rotatably engages a body-side part 202, e.g., as described above.

In this example, the tube-side part 204 includes a rotator 208 that is integrally formed with a grip 206B (which may, e.g., be or include a handle extending radially outwards from the rotator 208 as shown in Figs. 7a, 7b). A kink protection/cover 206A covering the rotator 208 (e.g., except for the grip/handle 206B) and the proximal portion of the insertion tube 104 may be formed either as a separate piece or integrally with the rotator 208 and the grip 206B.

The body-side part 202 is integrally formed with the body 102. The body 102 includes a body cover (e.g., housing or casing) 102A formed as two corresponding halves (only one of which is shown in Figs. 7a, 7b), wherein the distal portion of the body cover 102A forms the body-side part 202. The body-side part 202 includes a pair of (e.g., U-shaped or annular) bearing structures 222C, which define a rotation axis for the tube-side part 204 that coincides with the axis of the insertion tube 104.

The tube-side part 204 includes a cylindrical extension 208A extending from the tube-side part 204 along the proximal direction into the body cover 102A (i.e., into the body-side part 202). The cylindrical extension 208A has an annular portion 222D that protrudes radially outward from the cylindrical extension 208A and is arranged between the pair of bearing structures 222C. A pair of deformable members 224 (e.g., O-rings) is arranged between the bearing structures 222C and the annular portion 222D with the deformable members 224 being axially compressed by axial bearing surfaces formed by the bearing structures 222C and the annular portion 222D (and extending perpendicular to the axis of the insertion tube 104). The body-side part 202 may additionally include an end stop or a pair of end stops (formed by the body cover 102A) that are to engage a corresponding counterpart on the tube-side part 202 (e.g., a projection on the annular portion 222D) to limit a range of rotation of the insertion tube 104.

The examples of the present disclosure disclosed herein only constitute specific examples for illustration purposes. The present invention can be implemented in various ways and with many modifications without altering the underlying basic properties. Therefore, the present invention is only defined by the claims as stated below.

## Claims

1. A coupling device (200) for rotatably coupling an insertion tube (104) to a body (102) of a surgical device (100, 300), the coupling device (200) comprising:
a body-side part (202) that is attached or attachable to the body (102) of the surgical device (100, 300); and
a tube-side part (204) that is attached or attachable to the insertion tube (104),
wherein the tube-side part (204) is configured to rotatably engage the body-side part (202) so as to allow for rotation of the insertion tube (104) relative to the body (102) of the surgical device (100, 300) and so as to retain the insertion tube (104) at one or more discrete rotational orientations relative to the body (102) of the surgical device (100, 300).

2. The coupling device (200) of claim 1, wherein the tube-side part (204) is configured to rotatably engage the body-side part (202) via a snap fit.

3. The coupling device (200) of claim 2, wherein one of the body-side part (202) and the tube-side part (204) comprises a cantilever (214) configured to snap into and/or onto a corresponding receiving structure (216), in particular a groove, on the other one of the body-side part (202) and the tube-side part (204) to axially retain the insertion tube (104) relative to the body (102) of the surgical device (100, 300).

4. The coupling device (200) of any one of the preceding claims, wherein one of the body-side part (202) and the tube-side part (204), in particular said receiving structure (216), comprises one or more protrusions (216A) and/or one or more depressions (204B) that are arranged along a circumference of the body-side part (202) and the tube-side part (204), respectively, and configured to engage a corresponding counterpart (202B, 214A) on the other one of the body-side part (202) and the tube-side part (204), in particular on the cantilever (214), to retain the insertion tube (104) at said one or more discrete rotational orientations relative to the body (102) of the surgical device (100, 300).

5. The coupling device (200) of claim 4, wherein said protrusions (216A) and/or depressions (204B) form a toothed feature (216B) defining a plurality of discrete rotational orientation for the insertion tube (104) relative to the body of the surgical device (100, 300).

6. The coupling device (200) of any one of the preceding claims, further comprising one or more deformable members (224) that are arranged between the body-side part (202) and the tube-side part (204) and configured to generate friction resisting the rotation of the insertion tube (104) relative to the body (102) of the surgical device (100, 300).

7. The coupling device (200) of claim 6, wherein one or more of said deformable members (224) are arranged between axial bearing surfaces (222A, 222B) of the body-side part (202) and of the tube-side part (204) extending perpendicular or substantially perpendicular to an axial direction (Z) of the insertion tube (104).

8. The coupling device (200) of claim 6 or 7, wherein one or more of said deformable members (224) are arranged between radial bearing surfaces (202A, 204A, 212) of the body-side part (202) and of the tube-side part (204) extending parallel or substantially parallel to the axial direction (Z) of the insertion tube (104).

9. The coupling device (200) of any one of the preceding claims, wherein said coupling device (200) comprises one or more end stops (220) configured to limit a range of rotation of the insertion tube (104) relative to the body (102) of the surgical device (100, 300).

10. The coupling device (200) of any one of the preceding claims, wherein the body-side part (202) is integrally formed with the body (102) of the surgical device (100, 300).

11. The coupling device (200) of any one of the preceding claims, wherein said tube-side part (204) comprises a kink protection (206A) for preventing kinking of the insertion tube (104) at the body (102) of the surgical device (100, 300).

12. The coupling device (200) of claim 11, wherein said tube-side part (204) comprises a grip (206B) for rotating the insertion tube (104) relative to the body (102) of the surgical device (100, 300) and said grip (206B) is integrally formed with said kink protection (206A) as a single piece.

13. The coupling device (200) of any one of the preceding claims, wherein the tube-side part (204) comprises:
a rotator (208) that is attached or attachable to the insertion tube (104) and configured to rotatably engage the body-side part (202); and
a cover (206) arranged or arrangeable over the rotator (208) for covering and operating the rotator (208).

14. The coupling device (200) of claim 13 and one of claims 11 and 12, wherein the cover (206) forms one or both of said kink protection (206A) and said grip (206B).

15. A surgical device (100, 300) comprising an interface and/or control body (102), an insertion tube (104) and a coupling device (200) according to any one of the preceding claims for rotatably coupling the insertion tube (104) to the interface and/or control body (102), wherein optionally the surgical device (100, 300) is an endoscope (300), in particular a bronchoscope, a uteroscope, a colonoscope, a duodenoscope or a gastrointestinal endoscope.
